# EUROPÄISCHE PATENTSCHRIFT

(11) **EP 1 523 342 B1**
(45) Veröffentlichungstag und Bekanntmachung des Hinweises auf die Patenterteilung: **22.07.2009**
(21) Anmeldenummer: 03747406.1
(22) Anmeldetag: 07.04.2003
(51) Int. Cl.: A61L 27/12, A61L 27/56, A61L 31/12, A61L 31/14, A61L 27/46

(54) **STRUKTURIERTE KOMPOSITE ALS MATRIX (SCAFFOLD) FUR DAS TISSUE ENGINEERING VON KNOCHEN**
STRUCTURED COMPOSITES AS A MATRIX (SCAFFOLD) FOR THE TISSUE ENGINEERING OF BONES
COMPOSITES STRUCTURES EN TANT QUE MATRICE POUR LA PRODUCTION DE TISSUS OSSEUX PAR INGENIERIE GENETIQUE

(30) Priorität: 29.04.2002 DE 10219183
(43) Veröffentlichungstag der Anmeldung: 20.04.2005
(73) Patentinhaber: Biomet Deutschland GmbH, 14167 Berlin (DE)
(72) Erfinder: NIES, Berthold, 64407 Fränkisch-Crumbach (DE); JESCHKE, Brigitte, 65779 Kelkheim (DE); SCHAFFNER, Patricia, 64347 Griesheim (DE)
(74) Vertreter: Gross, Felix
(86) Internationale Anmeldenummer: PCT/EP2003/003582
(87) Internationale Veröffentlichungsnummer: WO 2003/092760

(56) Entgegenhaltungen:
- EP-A- 0 197 693
- EP-A- 0 519 293
- DE-U- 29 922 585

## Beschreibung

Die Erfindung beschreibt ein strukturiertes Komposit als Träger für das Tissue Engineering und Implantatmaterial von Knochen (in situ und in vitro) bestehend aus einer Schüttung von porösen Calciumphosphat-Granulaten mit einer Korngröße von 0.5 bis 10 mm mit Makro- und Mikroporen, wobei die Makroporen einen mittleren Durchmesser von 50 bis 500 µm haben und durch ein bioverträgliches Bindemittel gebunden sind.

Synthetischer Ersatz für Eigenknochen oder Spenderknochen gewinnt zunehmend an Bedeutung, weil einerseits die Qualität der entwickelten Materialien zunehmend die Anforderungen der Kunden erfüllt und andererseits die mit den biologischen Materialien verbundenen Nachteile vermieden werden, wie insbesondere eingeschränkte Verfügbarkeit, Entnahmeprobleme beim Eigenknochen, Risiko der Krankheitsübertragung beim Spenderknochen.
Um die biologische Wertigkeit der synthetischen Materialien zu erhöhen, gibt es die Ansätze, den Knochenersatz mit rekombinanten Wachstumsfaktoren, autologen Wachstumsfaktoren (Thrombozyten-Präparationen), Adhäsionsfaktoren (RGD-Peptide) und/oder autologen Zellpräparationen (Knochenmarkaspirat) aufzuwerten.

Um die gewünschten Eigenschaften von optimierten Knochenersatzmaterialien zu erhalten, gibt es vor allem zwei Entwicklungsschwerpunkte, einmal die mechanische Optimierung für lasttragende Anwendungen bzw. strukturelle Implantate und zum anderen die biologische Optimierung mit dem Ziel einer möglichst schnellen und vollständigen Regeneration von vollwertigen Knochengewebe (während dabei die mechanische Funktion von Metallimplantaten übernommen wird).

Die Zielrichtung für biologisch optimierte Knochen-Regenerationsprodukte war lange Zeit auf die Entwicklung rekombinanter Knochenwachstumsfaktoren (insbesondere BMP2 und OP1/BMP7) und deren Kombination mit verschiedenen Trägermaterialien und funktionellen Implantaten konzentriert, wobei in den meisten Fällen allerdings die Entwicklung geeigneter Trägerstrukturen mit geringerer Priorität verfolgt wurde als die des rekombinanten Proteins.
In letzter Zeit hat sich die Zielrichtung mehr und mehr verschoben, u.a. auch deshalb, weil die rekombinanten Wachstumsfaktoren die hohen Erwartungen in klinischen Studien nicht erfüllen konnten.

Attraktive Alternativen sind vor allem in der Verwendung autologer Faktoren wie insbesondere Thrombozyten-Präparationen und den osteogen wirkenden Knochenmarksaspiranten zu sehen. Die Gewinnung dieser Komponenten ist im Vergleich zur Entnahme von eigenem Knochen relativ unaufwendig und in der Menge kaum begrenzt. Zudem werden diese Faktoren anders als Beckenkamm-Spongiosa relativ schnell regeneriert und stehen damit für spätere Eingriffe wieder zur Verfügung.

Bei der Verwendung dieser autologen Faktoren kommt der Auswahl der Trägermaterialien eine entscheidende Bedeutung zu. Ein idealer Träger für viele Einsatzgebiete von Materialien, die die Knochenregeneration fördern, hat vor allem folgende Eigenschaften:
- Alle Komponenten sind biokompatibel
- Alle Komponenten sind osteokonduktiv, d.h. sie bilden eine Leitschiene für den neu zu bildenden Knochen.
- Alle Komponenten sind biodegradierbar, vorzugsweise durch zelluläre Prozesse, die auch für den Knochenumbau verantwortlich sind.
- Falls nicht oder nur eingeschränkt biodegradierbar, sollten die Komponenten fest in die gebildete Knochensubstanz eingebaut werden.
- Poröse Struktur mit interkonnektierenden Makroporensystem zum Einwachsen von Kapillaren in die Matrix
- Hohe Gesamtporosität zur Aufnahme der autologen Faktoren
- Ausreichende mechanische Eigenschaften, so dass das Porensystem auch bei Druckbelastung durch anliegendes Bindegewebe erhalten bleibt und für das Einwachsen von ortständigen Gewebe verfügbar ist.
- Flexibilität des Trägermaterials zur guten Anpassung an die Implantatkontur und allgemein gute Handhabbarkeit.
- Geringe Immunogenität.

Ein solches ideales Trägermaterial ist trotz des vielfältigen Angebots an Knochenersatzmaterialien derzeit nicht verfügbar. Vor allem die Kombination von hoher Porosität und Flexibilität bei gleichzeitiger Druckfestigkeit ist bisher nicht realisiert.

Aus der WO 97/14376 (Orquest Inc.) ist eine poröse biodegradierbare Matrix als Knochenersatz bekannt, die 7 bis 14 Tage nach der Implantation anhält. Das Komposit bildet ein Netzwerk aus unlöslichen biopolymeren Fibrillen, Calciumphosphatmineralien und u.a. 30 bis 80 Gew. % mineralisiertes Kollagen. Die Partikelgröße des Calciumphosphatminerals beträgt 5 µm oder weniger.

Die EP-197 693 und EP-270 254 (Collagen Corp.) beschreiben eine biologisch verträgliche Collagen/Calciumphosphat-Mischung, die im wesentlichen aus 60 bis 98 Gew.% Calciumphosphatmineral unter Beimengung von 2 bis 40 Gew. % eines rekonstituierten Atelopeptidfibrillärkollagens besteht. Die Partikelgröße des Calciumphosphats beträgt 100 bis 2000 µm.

Aufgabe der vorliegenden Erfindung war es, auf der Basis bekannter und bewährter Komponenten ein Material mit den oben genannten Eigenschaften zu realisieren.

Es wurde nun überraschend gefunden, dass sich poröse Granulate aus Calciumphosphaten mit bioverträglichen Bindemitteln, vorzugsweise Kollagensuspensionen, mischen lassen und dass durch Gefriertrocknung aus diesen Suspensionen Komposite hergestellt werden können, die den Anforderungen entsprechen.

Gegenstand der vorliegenden Erfindung ist somit ein strukturiertes Komposit als Träger für das Tissue Engineering und Implantatmaterial von Knochen bestehend aus einer Schüttung von porösen Calciumphosphat-Granulaten, **dadurch gekennzeichnet, dass** das Granulat eine Korngröße von 0.5 bis 10 mm mit Makro- und Mikroporen besitzt, wobei die Makroporen einen mittleren Durchmesser von 50 bis 500 µm und die Mikroporen einen mittleren Durchmesser von 0.5 bis 5 µm haben und durch ein bioverträgliches Bindemittel, das aus Kollagen oder Hyaluronsäure besteht und auch vernetzt vorliegen kann, gebunden sind.

In diesem Komposit sind die einzelnen Granula gleichmäßig z.B. in einem Kollagenvlies verteilt. Das Porensystem ist sowohl in den Granula als auch im Kollagenvlies realisiert. Durch die Druckfestigkeit des Granulats lässt sich das Komposit nicht komprimieren, dies ist vor allem in Anwendungen entscheidend, in denen das Material auf ein Knochenlager aufgelegt wird und mit einer Seite an das Weichgewebe grenzt, damit also dem Weichgewebedruck ausgesetzt ist. Solche Fälle sind vor allem im Bereich der Wirbeisäutenchirurgie sehr häufig.

Erfindungsgemäß besteht das Komposit aus einem porösen Calciumphosphat-Granulat mit einer Größe der Granulatkörner von 0.5 bis 10 mm, vorzugsweise 1 bis 4 mm. Bevorzugt ist weiterhin eine gleichmäßige Größenverteilung des Granulats im jeweiligen Komposit z.B. 2.0 bis 3.2 mm Korngröße.
Das Granulat besteht aus einem der für Knochenersatz gebräuchlichen Calciumphosphate. Vorzugsweise wird Endobon^{®} oder Calcibon^{®} Granulat verwendet. Es können aber auch Glaskeramiken eingesetzt werden. Insbesondere bei grobkörnigen Granulaten, d.h. mit einer Korngröße ab 2 mm auswärts, sollte die Porosität der Einzelkörner interkonnektierend sein, d.h. die bestehenden Poren innerhalb der Einzelkörner müssen miteinander in Verbindung stehen.

Das Komposit besteht vorzugsweise aus 0.2 bis 10 Gew. %., noch bevorzugter aus 0.2 bis 2 Gew.% Bindemittel, bezogen auf das Komposit.

Das Bindemittel hat vor allem die Funktion, die Granulatkörner miteinander zu verbinden und wie ein Schwamm die autologen Faktoren aufzunehmen, so dass sie für die Anwendung optimal lokalisiert werden können. Es findet keine starke Aufquellung statt, so dass die Handhabbarkeit erleichtert wird. Es dient weiterhin für die Vereinfachung der Handhabung des Granulatmaterials indem es eine Dislokalisation der Einzelkörner während der Operation und in den ersten Tagen danach verhindert.

Als Bindemittel werden vor allem solche Kollagene verwendet, die auch schon im Stand der Technik in oder als Medizinprodukte eingesetzt werden, insbesondere Typ-I-Kollagene und Mischungen aus Typ I und III. Die Herkunft kann sowohl tierisch, human oder rekombinant sein. Bevorzugt ist fibrilläres Kollagen nicht boviner, aber tierischer Herkunft.

Die Komposite können mit auto logen Faktoren wie Wachstumsfakoren, Thrombozytenpräparationen und/oder autologe Zellpräparationen und/oder Zellextrakte/Proteinpräparationen beladen werden und diese dann auch an den Knochen abgeben werden.
Bevorzugt ist auch, dass das Komposit mit rekombinanten Faktoren oder deren Teilstücke wie Wachstumsfaktoren, Adhäsionsproteine und/oder Adhäsionspeptiden beladen ist. Weiterhin bevorzugt ist die Beladung mit Antibiotika.
Denkbar ist ebenfalls der Einsatz von vernetzter Gelatine anstelle von Kollagen.

Gegenstand der vorliegenden Erfindung ist auch ein Verfahren zur Herstellung eines strukturierten Komposits als Träger für das Tissue Engineering und Implantatmaterial von Knochen, gekennzeichnet durch folgende Merkmale:
- Mischen einer Lösung oder Suspension von Bindemittel mit Calciumphosphat-Granulat mit einer Korngröße von 0.5 bis 10 mm mit Makro- und Mikroporen, wobei die Makroporen einen mittleren Durchmesser von 50 bis 500 µm haben;
- anschließend tiefgefrieren und auftauen sowie Gefriertrocknung des Gemisches unter Standardbedingungen.

Bevorzugt ist auch eine nachfolgende chemische Vernetzung.

Gegenstand der vorliegenden Erfindung ist auch die Verwendung des erfindungsgemäßen strukturierten Komposits zur Herstellung eines Füllmaterials im Bereich der Wirbelsäulen-, Kiefer- oder Gesichtschirurgie.
Die Erfindung wird nachfolgend anhand von einem Ausführungsbeispiel näher beschrieben und erläutert.

### Ausführungsbeispiel:

### 1. Herstellung

Das Komposit kann in unterschiedlichen Größen hergestellt werden. Aus einem Gemisch von 30 g Collagen-Suspension mit 7g Calciumphosphat-Granulat wird ein Komposit mit der Größe 8 x 2 cm mit 0.7 cm Dicke hergestellt. Das Gemisch wird über Nacht tiefgefroren, dann kurzzeitig aufgetaut. Im Anschluss findet die Gefriertrocknung des Gemisches unter Standardbedingungen statt. Standardbedingungen bedeutet: Einfrierphase (-40° C, 1-3 Std.), Erwärmungsphase (0° C, 1-3 Std.), Trocknungsphase (20° C, 12-18 Std.)
Das erhaltene Produkt enthält 98 bis 99 Gew. % Calciumphosphat und 1 bis 2 Gew. % Collagen.

### 2. Mischung mit Blut:

Das erhaltene Komposit zeigt hervorragende Eigenschaften bei der Benetzung mit Blut. Es saugt das Blut vollständig auf. Es zeigt sich keine Quellung oder Verformung des Komposits.

### 3. Stabilität

Eine Stabilität in Puffer kann über zwölf Tage nachgewiesen werden. Innerhalb dieser zwölf Tage bleibt das Komposit kompakt und die Granula werden zusammengehalten.

## Patentansprüche

1. Strukturiertes Komposit als Träger für das Tissue Engineering und Implantatmaterial von Knochen bestehend aus einer Schüttung von porösen Calciumphosphat-Granulaten, **dadurch gekennzeichnet, dass** das Granulat eine Korngröße von 0,5 bis 10 mm mit Makro- und Mikroporen besitzt, wobei die Makroporen einen mittleren Durchmesser von 50 bis 500 µm und die Mikroporen einen mittleren Durchmesser von 0,5 bis 5 µm haben, gebunden durch ein bioverträgliches Bindemittel, das Kollagen, vernetzte Gelatine oder Hyaluronsäure ist.

2. Komposit nach Anspruch 1, **dadurch gekennzeichnet, dass** die Größe der Granulatkörner 1 bis 4 mm beträgt.

3. Komposit nach einem der Ansprüche 1 oder 2, **dadurch gekennzeichnet, dass** es aus 0,2 bis 10 Gew. % Bindemittel, bezogen auf das Komposit, besteht.

4. Komposit nach einem der Ansprüche 1 oder 2, **dadurch gekennzeichnet, dass** es aus 0,2 bis 2 Gew. % Bindemittel, bezogen auf das Komposit, besteht.

5. Komposit nach einem der Ansprüche 1 bis 4, **dadurch gekennzeichnet, dass** das Bindemittel Kollagen aus Typ-I-Kollagen oder Mischungen aus Typ I und III besteht.

6. Komposit nach einem der Ansprüche 1 bis 5, **dadurch gekennzeichnet, dass** das Bindemittel vernetzt wird.

7. Komposit nach einem der Ansprüche 1 bis 6, **dadurch gekennzeichnet, dass** es mit autologen Faktoren wie Wachstumsfaktoren, Thrombozytenpräparationen und/oder autologen Zellpräparationen und/oder Zellextrakten/Proteinpräparationen beladen ist.

8. Komposit nach einem der Ansprüche 1 bis 6, **dadurch gekennzeichnet, dass** es mit rekombinanten Faktoren oder deren Teilstücken wie Wachstumsfaktoren, Adhäsionsproteinen und/oder Adhäsionspeptiden beladen ist.

9. Komposit nach einem der Ansprüche 1 bis 6, **dadurch gekennzeichnet, dass** es mit Antibiotika beladen ist.

10. Verfahren zur Herstellung eines strukturierten Komposits als Träger für das Tissue Engineering und Implantatmaterial von Knochen, **gekennzeichnet durch** folgende Merkmale:
Mischen einer Lösung oder Suspension von Bindemittel mit Calciumphosphat-Granulat mit einer Korngröße von 0,5 bis 10 mm mit Makro- und Mikroporen, wobei die Makroporen einen mittleren Durchmesser von 50 bis 500 µm haben;
anschließend tiefgefrieren und auftauen sowie Gefriertrocknung des Gemisches unter Standardbedingungen.

11. Verfahren nach Anspruch 10, **dadurch gekennzeichnet, dass** das Bindemittel nachfolgend chemisch vernetzt wird.

12. Verwendung eines strukturierten Komposits nach einem der Ansprüche 1 bis 9 zur Herstellung eines Füllmaterials im Bereich der Wirbelsäulen-, Kiefer- oder Gesichtschirurgie.

## Claims

1. Structured composite as a carrier for tissue engineering and implant material of bones consisting of a packed bed of porous calcium phosphate granules, **characterized in that** the granules have a grain size of from 0.5 to 10 mm with macro- and micropores, wherein the macropores have a mean diameter of from 50 to 500 µm and the micropores have a mean diameter of from 0.5 to 5 µm, bound by a biocompatible binder which is collagen, cross-linked gelatine or hyaluronic acid.

2. Composite according to claim 1, **characterized in that** the size of the grains of the granules is 1 to 4 mm.

3. Composite according to any of claims 1 or 2, **characterized in that** it consists of from 0.2 to 10 percent by weight, based on the composite, of binder.

4. Composite according to any of claims 1 or 2, **characterized in that** it consists of from 0.2 to 2 percent by weight, based on the composite, of binder.

5. Composite according to any of claims 1 to 4, **characterized in that** the binder collagen consists of type I collagen or of mixtures of type I and type III.

6. Composite according to any of claims 1 to 5, **characterized in that** the binder is being cross-linked.

7. Composite according to any of claims 1 to 6, **characterized in that** it is loaded with autologous factors like growth factors, thrombocyte preparations and/or autologous cell preparations and/or cell extracts/protein preparations.

8. Composite according to any of claims 1 to 6, **characterized in that** it is loaded with recombinant factors or their fragments like growth factors, adhesion proteins and/or adhesion peptides.

9. Composite according to any of claims 1 to 6, **characterized in that** it is loaded with antibiotics.

10. Method for preparing a structured composite as carrier for tissue engineering and implant material of bones, **characterized by** following features:
mixing a solution or suspension of binder with calcium phosphate granules having a grain size of from 0.5 to 10 mm having macro- and micropores, wherein the macropores have a mean diameter of from 50 to 500 µm;
subsequently deep-freezing and thawing as well as lyophilizing the mixture under standard conditions.

11. Method according to claim 10, **characterized in that** the binder is subsequently chemically cross-linked.

12. Use of a structured composite according to any of claims 1 to 9 for the preparation of a filling material in the area of spinal, oral or facial surgery.

## Revendications

1. Composite structuré en tant que porteur pour la ingénierie tissulaire et matériau d'implantation d'os, consistant en un remblai des granules poreux de phosphate de calcium, **caractérisé en ce que** les granules ont une taille de grain de 0,5 à 10 mm avec de pores macros et micros, où les pores macros ont un diamètre médian de 50 à 500 µm et les pores micros ont un diamètre médian de 0,5 à 5 µm, attaché par un liant biocompatible, qui est collagène, gélatine interconnectée ou acide hyaluronique.

2. Composite selon la revendication 1, **caractérisé en ce que** la taille des grains des granules est 1 à 4 mm.

3. Composite selon l'une quelconque des revendications 1 ou 2, **caractérisé en ce qu'**il consiste en 0,2 à 10 % en poids, relatif au composite, du liant.

4. Composite selon l'une quelconque des revendications 1 ou 2, **caractérisé en ce qu'**il consiste en 0,2 à 2 % en poids, relatif au composite, du liant.

5. Composite selon l'une quelconque des revendications 1 à 4, **caractérisé en ce que** le liant collagène consiste en collagène de type I ou en un mélange de type I et III.

6. Composite selon l'une quelconque des revendications 1 à 5, **caractérisé en ce que** le liant est fait interconnecté.

7. Composite selon l'une quelconque des revendications 1 à 6, **caractérisé en ce qu'**il est chargé de facteurs autologues comme des facteurs croissances, des préparations de thrombocytes et/ou des préparations autologues de cellules et/ou des extraits cellulaires/des préparations de protéine.

8. Composite selon l'une quelconque des revendications 1 à 6, **caractérisé en ce qu'**il est chargé de facteurs recombinants ou leurs fragments comme des facteurs croissance, des protéines d'adhérence et/ou des peptides d'adhérence.

9. Composite selon l'une quelconque des revendications 1 à 6, **caractérisé en ce qu'**il est chargé d'antibiotiques.

10. Méthode pour la production d'un composite structuré en tant que support pour la ingénierie tissulaire et matériau d'implantation d'os, **caractérisée par** les caractéristiques subséquentes :
mélanger une solution ou une suspension d'un liant avec granules de phosphate de calcium ayant une taille de grain de 0,5 à 10 mm ayant de pores macros et micros, où les pores macros ont un diamètre médian de 50 à 500 µm ;
ensuite congeler et décongeler ainsi que lyophiliser le mélange sur des conditions standard.

11. Méthode selon la revendication 10, **caractérisée en ce que** le liant est fait ensuite interconnecté par voie chimique.

12. Utilisation d'un composite structuré selon l'une quelconque des revendications 1 à 9 pour la production d'une masse de remplissage dans la branche de la chirurgie de rachis, maxillaire ou faciale.
